# EUROPEAN PATENT SPECIFICATION

(11) **EP 1 505 965 B1**
(45) Date of publication and mention of the grant of the patent: **02.08.2006**
(21) Application number: 03749892.0
(22) Date of filing: 13.05.2003
(51) Int. Cl.: A61K 31/41, A61P 7/02

(54) **USE OF THE METABOLITE OF VALSARTAN TO INHIBIT PLATELET AGGREGATION**
VERWENDUNG DES METABOLITS VON VALSARTAN ZUR HEMMUNG DER THROMBOZYTENAGGREGATION
UTILISATION DU METABOLITE DE VALSARTAN POUR INHIBER L'AGREGATION PLAQUETTAIRE

(30) Priority: 14.05.2002 US 380373 P; 11.07.2002 US 395014 P
(43) Date of publication of application: 16.02.2005
(73) Proprietor: Novartis AG, 4056 Basel (CH); Novartis Pharma GmbH, 1230 Wien (AT)
(72) Inventor: MALININ, Alex, Cockeysville, MD 21030 (US); SEREBRUANY, Victor, L., West Friendship, MD 21794 (US); WEBB, Randy, Lee, Flemington, NJ 08822 (US)
(74) Representative: Crawley, Patrick Edward
(86) International application number: PCT/EP2003/004997
(87) International publication number: WO 2003/094915

(56) References cited:
- EP-A- 1 197 226
- WO-A-01/82858
- WO-A-01/97805
- MONTON MERCEDES ET AL: "Comparative effects of angiotensin II AT-1-type receptor antagonists in vitro on human platelet activation." JOURNAL OF CARDIOVASCULAR PHARMACOLOGY, vol. 35, no. 6, June 2000 (2000-06), pages 906-913, XP009015828 ISSN: 0160-2446
- CHLOPICKI S ET AL: "Antiplatelet action of losartan involves TXA2 receptor antagonism but not TXA2 synthase inhibition." JOURNAL OF PHYSIOLOGY AND PHARMACOLOGY, vol. 51, no. 4 Part 1, December 2000 (2000-12), pages 715-722, XP002252057 ISSN: 0867-5910
- BUCZKO W ET AL: "Studies on the antithrombotic action of AT1 receptor antagonists" MEDICAL SCIENCE MONITOR 2001 POLAND, vol. 7, no. 4, 2001, pages 600-605, XP002252058 ISSN: 1234-1010
- GUERRA-CUESTA JOSE I ET AL: "Effect of losartan on human platelet activation." JOURNAL OF HYPERTENSION, vol. 17, no. 3, March 1999 (1999-03), pages 447-452, XP009015995 ISSN: 0263-6352
- BURNIER M M ET AL: "Angiotensin II receptor antagonists" LANCET, XX, XX, vol. 355, no. 9204, 19 February 2000 (2000-02-19), pages 637-645, XP004263093 ISSN: 0140-6736
- WALDMEIER F ET AL: "Pharmacokinetics, disposition and biotransformation of (14C)-radiolabelled valsartan in healthy male volunteers after a single oral dose." XENOBIOTICA, vol. 27, no. 1, 1997, pages 59-71, XP009015738 ISSN: 0049-8254 cited in the application
- SEREBRUANY VICTOR L ET AL: "Effects of valsartan and valeryl 4-hydroxy valsartan on human platelets: A possible missing link to explain reduction of acute vascular events with angiotensin II receptor blockers." JOURNAL OF THE AMERICAN COLLEGE OF CARDIOLOGY, vol. 41, no. 6 Supplement A, 19 March 2003 (2003-03-19), page 310A XP009015745 52nd Annual Scientific Session of the American College of Cardiology;Chicago, IL, USA; March 30-April 02, 2003 ISSN: 0735-1097
- KALINOWSKI LESZEK ET AL: "Angiotensin II AT1 receptor antagonists inhibit platelet adhesion and aggregation by nitric oxide release." HYPERTENSION (BALTIMORE), vol. 40, no. 4, October 2002 (2002-10), pages 521-527, XP009015813 October, 2002 ISSN: 0194-911X

## Description

### BACKGROUND OF THE INVENTION

Valsartan selectively blocks the binding of angiotensin II to the A_{T1} receptor causing vasodilatation, and diminishes aldosterone secretion. Recent clinical studies revealed additional benefits of Valsartan in a cohort of patients after acute vascular events. Considering that platelet activation plays a key role in the pathogenesis of coronary and cerebrovascular occlusion, and that A_{T1} receptors are present on the platelet surface the *in vitro* effects of Valsartan and its major liver metabolite, valeryl 4-hydroxy valsartan on platelets in subjects with multiple risk factors for vascular disease was assessed.

### SUMMARY OF THE INVENTION

In one embodiment the present invention relates to the use of the metabolite valeryl 4-hydroxy valsartan for the preparation of a medicament of inhibiting platelet aggregation, optionally in the presence of a pharmaceutically acceptable carrier.

In another aspect of the present invention there is provided the use of the metabolite valeryl 4-hydroxy valsartan for the preparation of a medicament for the treatment of conditions associated with platelet aggregation. Conditions associated with platelet aggregation include acute myocardial infraction, ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel, catheter thrombotic occlusion or reocclusion.

Another aspect of the present invention relates to the metabolite valeryl 4-hydroxy valsartan for use as a medicament.

### DETAILED DESCRIPTION OF THE PREFERRED EMBODIMENTS

AT₁-receptor antagonists (also called angiotensin II receptor antagonists) are understood to be those active ingredients which bind to the AT₁-receptor subtype of angiotensin II receptor but do not result in activation of the receptor. As a consequence of the inhibition of the AT₁ receptor, these antagonists can, for example, be employed as to prevent platelet aggregation and treat conditions associated therewith.

Valsartan, which is a AT 1 receptor antagonist has the formula (S)-N-(1-carboxy-2-methyl-prop-1-yl)-N-pentanoyl-N-[2;(1H-tetrazol-5-yl)biphenyl-4-yl-methyl]amine of formula (I) 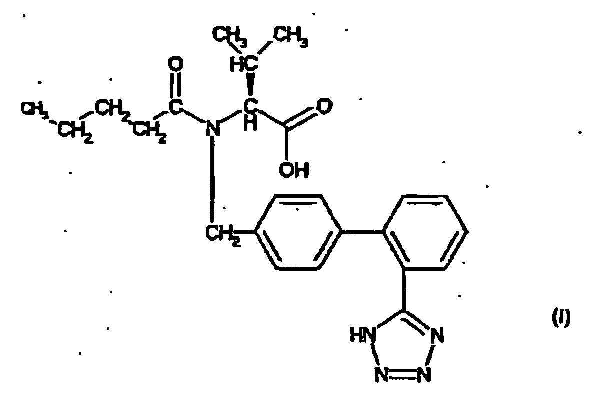 and is disclosed in EP 0443983 A and United States Patent 5,399,57B.

It has been surprisingly found that metabolites of ARBs namely, valeryl 4-hydroxy valsartan having the formula 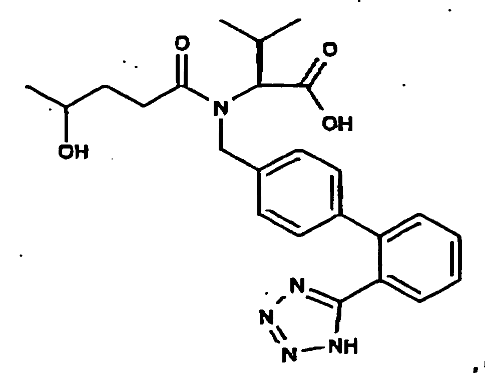 disclosed in Waldmeier F. et al., Xenobiotica, 1997. Vol. 27, No. 1, 59-71) significantly reduce platelet aggregation. This metabolite may be referred to herein as "the compound of the invention". The compound of the invention possesses one or more asymmetric centers. The resulting dlastereolsomers. enantiomers and geometric isomers are encompassed by the instant invention.
Depending on the choice of starting materials and methods, the compound may be in the form of one of the possible isomers or mixtures thereof, for example, as substantially pure geometric (cis or trans) isomers, optical isomers (antipodes), racemates. or mixtures thereof. The aforesald possible isomers or mixtures thereof are within the purview of this invention.

Any resulting mixtures of isomers can be separated on the basis of the physicochemical differences of the constituents, into the pure geometric or optical isomers. diastereoisomers, racemates, for example by chromatography and/or fractional crystallization.

Any resulting racemates of final products or intermediates can be resolved into the optical antipodes by known methods, e.g. by separation of the diastereolsomeric salts thereof, obtained with an optically active acid or base, and liberating the optically active acidic or basic compound. The carboxylic acid intermediates can thus be resolved into their optical antipodes e.g. by fractional crystallization of D- or L-(alpha-methylbenzylamine, cinchonidine. cinchonine. quinine, quinidine, ephedrine, dehydroabletylamine, brucine or strychnine}-salts. Racemic products can also be resolved by chiral chromatography. e.g. high pressure liquid chromatography using a chiral adsorbent.

Finally, the compound of the invention are either obtained in the free form, or as a salt thereof if salt forming groups are present.

Acidic compounds of the invention may be converted into salts with pharmaceutically acceptable bases, e.g. an aqueous alkali metal hydroxide, advantageously in the presence of an ethereal or alcoholic solvent, such as a lower alkanol From the solutions of the latter, the salts may be precipitated with ethers, e.g. diethyl ether. Resulting salts may be converted into the free compounds by treatment with acids. These or other salts can also be used for purification of the compounds obtained.

The Compound of the invention having basic groups can be converted into acid addition salts, especially pharmaceutically acceptable salts. These are formed, for example, with inorganic acids, such as mineral acids, for example sulfuric acid, a phosphoric or hydrohalic acid, or with organic carboxylic acids, such as (C₁-C₄)-alkanecarboxylic acids which, for example, are unsubstituted or substituted by halogen, for example acetic acid, such as saturated or unsaturated dicarboxylic acids, for example oxalic, succinic, maleic or fumaric acid, such as hydroxy-carboxylic acids, for example glycolic, lactic, malic, tartaric or citric acid, such as amino acids, for example aspartic or glutamic acid, or with organic sulfonic acids, such as (C₁-C₄)-alkyl-sulfonic acids (for example methanesulfonic acid) or arylsulfonic acids which are unsubstituted or substituted (for example by halogen). Preferred are salts formed with hydrochloric acid, methenesulfonic acid and maleic acid.

In view of the close relationship between the free compound and the compounds in the form of their salts, whenever a compound is referred to in this context, a corresponding salt is also intended, provided such is possible or appropriate under the circumstances.

The compound, including its salts, can also be obtained in the form of its hydrate, or include other solvents used for their crystallization.

The compound can be formulated into pharmaceutical compositions comprising a therapeutically effective amount of the compound of the invention and a pharmaceutically acceptable carrier. The pharmaceutical compositions used in the invention can be prepared in a manner known per so and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application. Typical oral formulations include tablets, capsules, syrups, elixirs and suspensions. Typical injectable formulations include solutions and suspensions. The pharmaceutical compositions may be employed for the treatment of conditions mediated by platelet aggregation, in particular, acute myocardial infarction, ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel catheter thrombotic occlusion or reocclusion.
The typical pharmaceutically acceptable carriers for use in the formulations described above are exemplified by: sugars such as lactose, sucrose, mannitol and sorbitol; starches such as comstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil: non-ionic, cationic and anionic surfactants; ethylene glycol polymers; betacyciodextrin; fatty alcohols; and hydrolyzed cereal solids, as well as other non-toxic compatible fillers, binders, disintegrants, buffers, preservatives, antioxidants, lubricants, flavoring agents, and the like commonly used in pharmaceutical formulations.
These pharmaceutical preparations are for enteral, such as oral, and also, rectal or parenteral, administration to homeotherms, with the preparations comprising the pharmacological active compound either alone or together with customary pharmaceutical auxiliary substances. For example, the pharmaceutical preparations consist of from 0.1 % to 90 %, preferably of from 1 % to 80 %, of the active compounds. Pharmaceutical preparations for enteral or parenteral administration are, for example, in unit dose forms, such as coated tablets, tablets, capsules or suppositories and also ampoules. These are prepared in a manner which is known per se, for example using conventional mixing, granulation, coating, solubulizing or lyophilizing processes. Thus, pharmaceutical preparations for oral use can be obtained by Combining the active compounds with solid excipients, if desired granulating a mixture which has been obtained, and, if required or necessary, processing the mixture or granulate into tablets or coated tablet cores after having added suitable auxiliary substances.

Valeryl 4-hydroxy valsartan may be combined with other therapeutic agents, e.g., each at an effective therapeutic dose as reported in the art. Such therapeutic agents include heparin, warfarin, t-PA, urokinase, streptokinase, aspirin, ticlopidine, clopidogrel, abciximab, eptifibatide and tirofiban, antihypertensive agents and anti-diabetics.
The dosage of the active compound can depend on a variety of factors, such as mode of administration, homeothermic species, age and/or individual condition.
Preferred dosages for the active ingredients according to the present invention are therapeutically effective dosages, especially those which are commercially available for valsartan.
Normally, in the case of oral administration of the compounds of the present invention, an approximate dally dose of from 0.1 mg to 360 mg is to be estimated e.g. for a patient of approximately 75 kg in weight.

In case of valerly 4-hydroxy valsartan, preferred dosage unit forms are, for example, tablets or capsules comprising e.g. for a mammal of 50 to 70 kg from 1 mg to 1000 mg, advantageously from 5 mg to 500 mg, even more advantageously from 20 mg to 320 mg, administered once a day.
The above doses encompass a therapeutically effective amount of the active ingredients of the present invention.
It has surprisingly been found that valeryl 4-hydroxy valsartan exhibits significant *in vitro* inhibition of human platelets.
The compound of the present invention inhibits platelet aggregation; and thus may be employed for the treatment of conditions mediated by platelet aggregation, in particular, acute myocardial infarction, Ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel, catheter thrombotic occlusion or reocclusion.

The invention furthermore also relates to a compound according to the invention for use in the prevention of, delay of progression of, treatment of a disease or condition mediated by platelet aggregation, in particular, acute myocardial infarction, ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel, catheter thrombotic occlusion or reocclusion.

The invention furthermore also relates to the use of a compound according to the invention for the manufacture of a medicament for the prevention, delay of progression or treatment of a disease and disorder mediated by platelet aggregation, in particular, acute myocardial infarction, ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel, catheter thrombotic occlusion or reocclusion.

The above-cited properties have been demonstrated by the following method:
Blood samples for platelet aggregation, flow cytometric studies, and cartridge-based platelet assay analyzers were obtained from 20 volunteers with known vascular risk factors. Study participants were excluded if they had a history of bleeding diathesis, history of stroke, major surgery or significant trauma in the past six months, and hypertension of more than 200/110 mm. None of them received aspirin or any other anti- platelet agents. All subjects underwent blood sampling after at least 30 minutes of rest and 2 or more hours of fasting. Blood was drawn between 8 and 10 a.m. in order to avoid any diurnal influence and sampled from an antecubital vein using a 21-gauge butterfly needle containing 3.8% sodium citrate (1:9 volume) after having discarded the first 1.5 ml of free running blood. Eleven Vacutainer tubes (4.5 ml) were collected for a total of 49 ml of whole blood - citrate mixture from each study participant. One tube was kept as an intemal control and was incubated with buffer solution. Five tubes were incubated with valsartan for 60 minutes at 37°C in order to achieve the final concentrations of the compound at 10nM, 100nM, 1µM, 10µM, and 100µM. The remaining five tubes were similarly incubated with increasing amounts of valeryl 4-hydroxy valsartan to achieve concentrations of 10nM, 100nM, 1µM, 10µM, and 100µM. The concentrations of valsartan and valeryl 4-hydroxy valsartan ranged from subtherapeutic to markedly supra-therapeutic plasma levels observed in patients undergoing valsartan therapy. Valsartan peak plasma concentration can reach as high as 7.5µM after a 160mg intake, while valeryl 4-hydroxy valsartan in plasma represents only 10% of valsartan levels. Fresh solutions of valsartan and valeryl 4-hydroxy valsartan were prepared ex tempore on the same morning the platelet studies were performed. To avoid possible observer bias, blood samples were coded and blinded. Sampling procedures, and platelet studies were performed by individuals unaware of the protocol.

### Platelet aggregation

### A. Platelet -rich plasma

The citrate and whole blood mixture were centrifuged at 1200g for 5 minutes in order to obtain platelet- rich plasma (PRP) which was kept at room temperature for use within 1 hour. Platelet counts were determined for each PRP sample with a Coulter Counter ZM (Coulter Co., Hialeah, FL). Platelet numbers were adjusted to 3.50 x 10 ⁸/ml with homologous platelet-poor plasma. Platelet aggregation (PA) was induced by 5 µM ADP, and 5 µM epinephrine. All agonists were obtained from Chronolog Corporation (Havertown, PA). Aggregation studies were performed using a 4 channel Chronolog Lumi-Aggregometer (model 560 - Ca). Aggregation was expressed as the maximum percentage of light transmittance change (% max) from the baseline at the end of the recording time, using platelet-poor plasma as a reference. Aggregation curves were recorded for 6 minutes and analyzed according to internationally established standards. Ruggeri ZM, Semin Hemat 1994; 31: 229 - 239.

### B. Whole blood

The methods are described in detail in Abbate R, et al., Amer J Clin Pathol 1986; 86: 91-96. Briefly, the whole blood citrate mixture was diluted 1:1 with 0.5 ml TBS, then swirled gently. The cuvette with the stirring bar was placed in the incubation well and allowed to warm to 37°C for 5 minutes. Then the sample was transferred to the assay well. An electrode was placed in the sample cuvette. Platelet aggregation was stimulated with 1 µg/ml collagen. Platelet aggregation studies were performed using a Chrono-Log Whole Blood Aggregometer using Aggrolink" software. Platelet aggregability was expressed as the change in electrical impedance and is expressed in ohms.

### Whole Blood Flow Cytometry

The expression of platelet receptors was determined by using the following monoclonal antibodies: CD31 (platelet endothelial cell adhesion molecule (PECAM-1), CD41 (glycoprotein [GP] IIb/IIIa, (IIb (3), CD42b (GP Ib), CD 51/CD61 ((v (3, or vitronectin receptor), CD62p (P-selectin), CD107a (lysosome associated membrane protein -1; LAMP-1), CD 107b (LAMP-2), CD151 (platelet/endothelial tetraspan antigen -3; PETA-3), and PAC-1 for fibrinogen-platelet (PharMingen, San Diego, CA). Platelet-leukocyte interactions were assessed by using dual antibodies for a pan-platelet marker (CD151), together with CD14, a monocyte/macrophage marker. The blood-citrate mixture (50 µl) was diluted with 450 µl Tris buffered saline (TBS) (10 mmol/L Tris, 0.15 mol/L sodium chloride) and mixed by gently inverting an Eppendorf tube 2 times. Five µl of the corresponding antibodies were then added to each solution and the samples were incubated for 30 minutes. After incubation, 400 µl of 2% buffered paraformaldehyde was added for fixation. The samples were analyzed by a Becton Dickinson FACScan flow cytometer set up to measure fluorescent light scatter, as described in Gurbel PA, et al., J Am Coll Cardiol. 1998;31:1466-1473. The data were collected in list mode files and then analyzed. P selectin was expressed as percent positive cells as described in Gurbel PA, et al, Am Heart J 2000; 139: 320-328. Other antigens were expressed as log mean fluorescence intensity.

### Cartridge-Based Platelet Analyzers

The platelet function analyzer (PFA-100', Dade Behring, Deerfield, IL) is a device that simulates changes in primary hemostasis after injury to a small vessel under flow conditions. Kundu SK, et al., Semin Thromb Hemost 1995;21 (Suppl 2):106-112. The time required to obtain occlusion of the aperture was digitally recorded as a measure of shear-induced platelet aggregation. Closure time determinations were performed in duplicate.
A rapid platelet-function assay cartridge test (RPFA-ASA, Ultegra(r) Accumetrics, Inc., San Diego, CA, USA), using polystyrene beads coated cartridges with lyophilized human fibrinogen-coated microparticles, and propyl gallat served as an agonist. The whole blood citrate mixture was added to the cartridge, and agglutination between platelets and coated beads was recorded. The data mirrored turbidometric platelet aggregation and reflected the degree of platelet prostaglandin blockade. Smith JW, et al., Circulation 1999;99:620-625. Ultegra(r) assays were performed in duplicate. An electronic quality control test was performed on each instrument every day of use prior to performing any subject samples.

### Statistical Analysis

All comparisons were calculated by Student's t-test to identify specific differences in platelet aggregation, results of Ultegra(r), Dade-PFA 100(tm), and receptor expression between baseline and post valsartan/valeryl 4-hydroxy valsartan incubation. The Mann-Whitney U test was used to analyze non-parametric data. Normally distributed data were expressed as mean ± SE, and skewed data as median (range). Probability values of p< 0.05 were regarded as statistically significant. Linear regression analysis was applied to normally distributed data for all study participants by using the SPSS v9.0 program (SPSS Inc. Chicago, Illinois) for statistical analysis.

### Platelet aggregation in platelet-rich plasma

Preincubation with escalating doses of valsartan resulted in inhibition of ADP - induced platelet aggregation only at a concentration of 100µM which exceeds the therapeutic level, and did not affect the epinephrine-induced platelet aggregation. In contrast, incubation with valeryl 4-hydroxy valsartan inhibited epinephrine-induced aggregation in concentration 1µM and 10µM, and did not have any effect on ADP-induced aggregation. Representative data for 5µM ADP and 5µM epinephrine-stimulated aggregation are shown at Table 1.

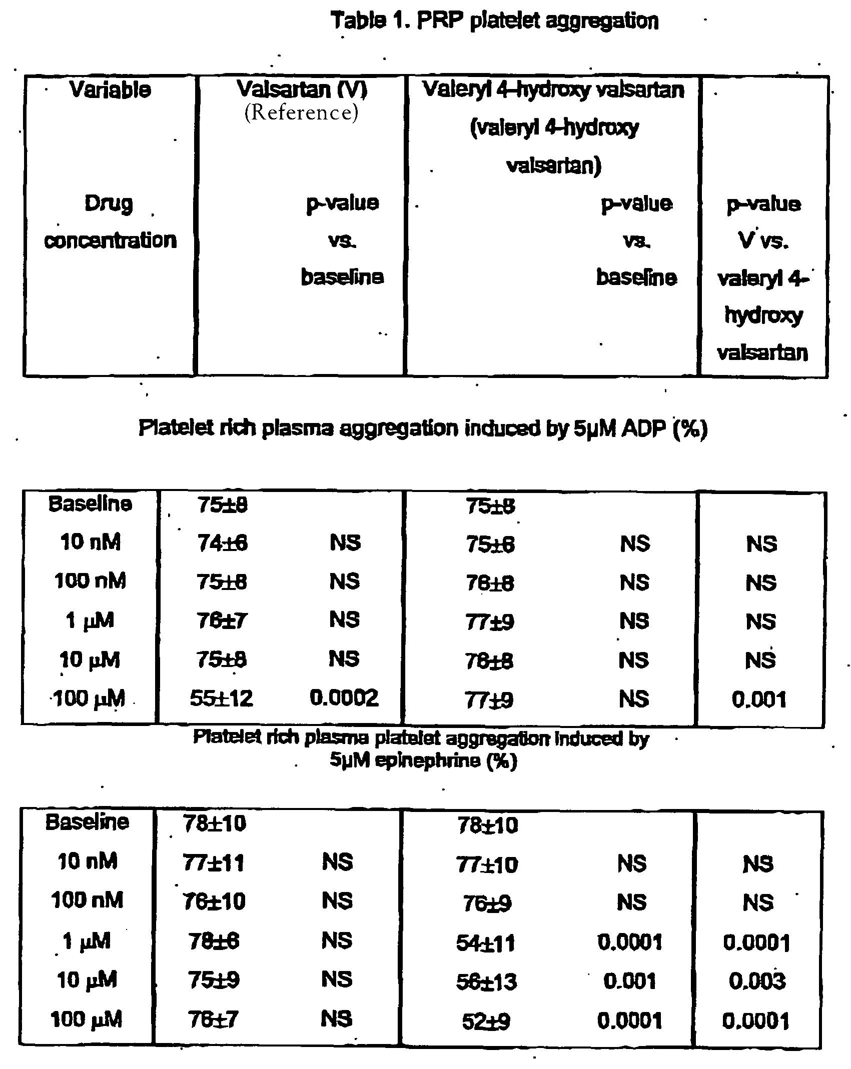

### Platelet aggregation in whole blood

Valsartan and valeryl 4-hydroxy valsartan inhibited aggregation of human platelets induced by 1 µM collagen in whole blood within the range of therapeutic activity. A dose dependent effect was observed for the both of compound, but preincubation with valeryl 4-hydroxy valsartan resulted in a significant reduction of platelet aggregability. The results of collagen - induced platelet aggregation are demonstrated by Table 2.

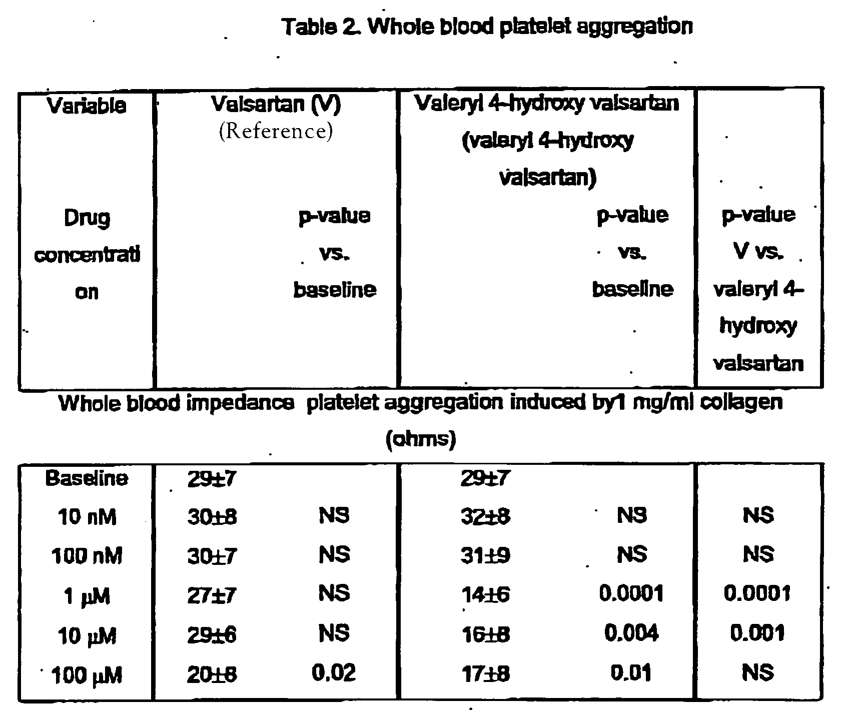

### Cartridge-based Platelet Function Analysers (PFA-100™ and Ultegre® )

A consistent dose-dependent delay of the closure time (PFA) as a reduction of platelet aggregation units (Ultegra) was observed, indicating platelet inhibition under high shear conditions. Similar to the whole blood aggregation and epinephrine-induced aggregation. valeryl 4-hydroxy valsartan had stronger antiptatelet properties than valsartan. Table 3 demonstrates a representative experiment with cartridge-based analyzers.

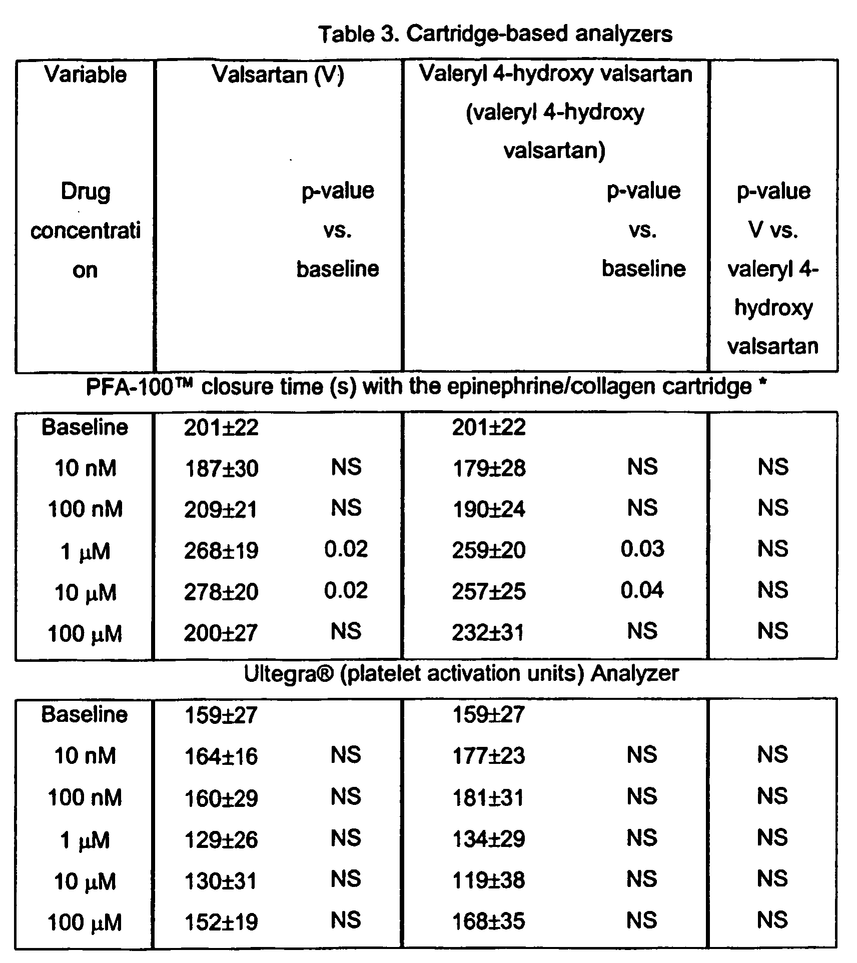

### Whole blood flow cytometry

Incubation with valsartan and valeryl 4-hydroxy valsartan slightly reduced the expression of GP IIb/IIIa (CD 41) and fibrinogen binding (PAC-1), and valeryl 4-hydroxy valsartan achieved this effect at therapeutic concentrations, whereas valsartan decreased expression of CD41 only at a high dose. Both agents significantly diminished concentration of P-selectin on platelet surface, and moderately reduced the expression of vitronectin receptors, even this effect did not have statistical power to show d!tference between valsartan and valeryl 4-hydoxy valsartan. The Incubation with vatsanan and valeryl 4-nydroxy vaisanan was not associated with any effect on PECAM-1 (CD31); GP lb (CD42) and LAMP-2 (CD107b), PETA-3 (CD151), and platelet-leukocyte mlcropartides (CD151+14) The results of flow cyclometry are presented on Table 4.

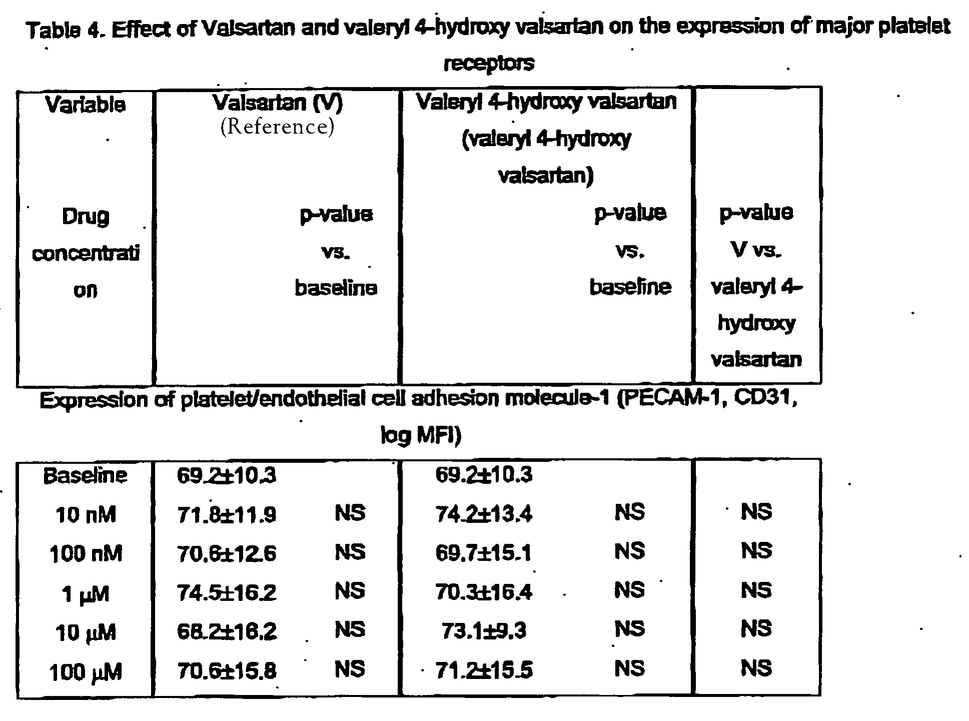

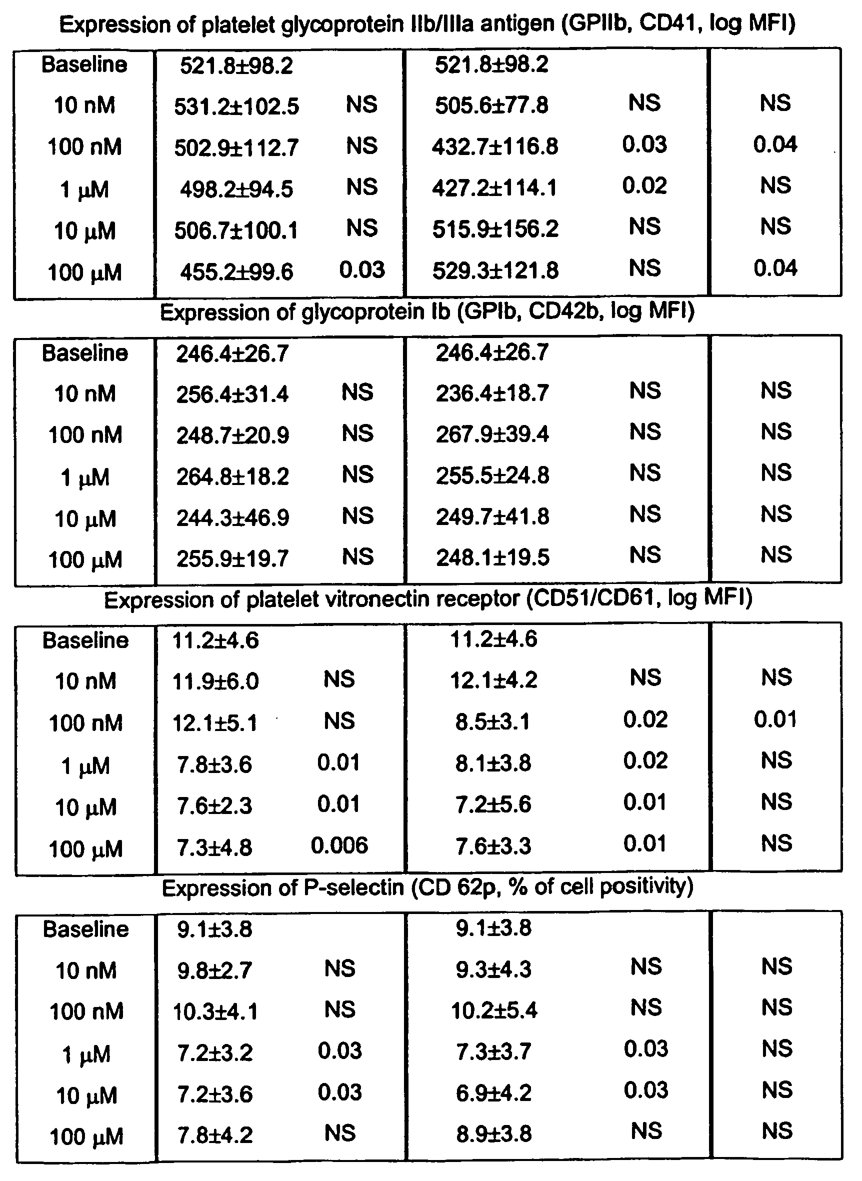

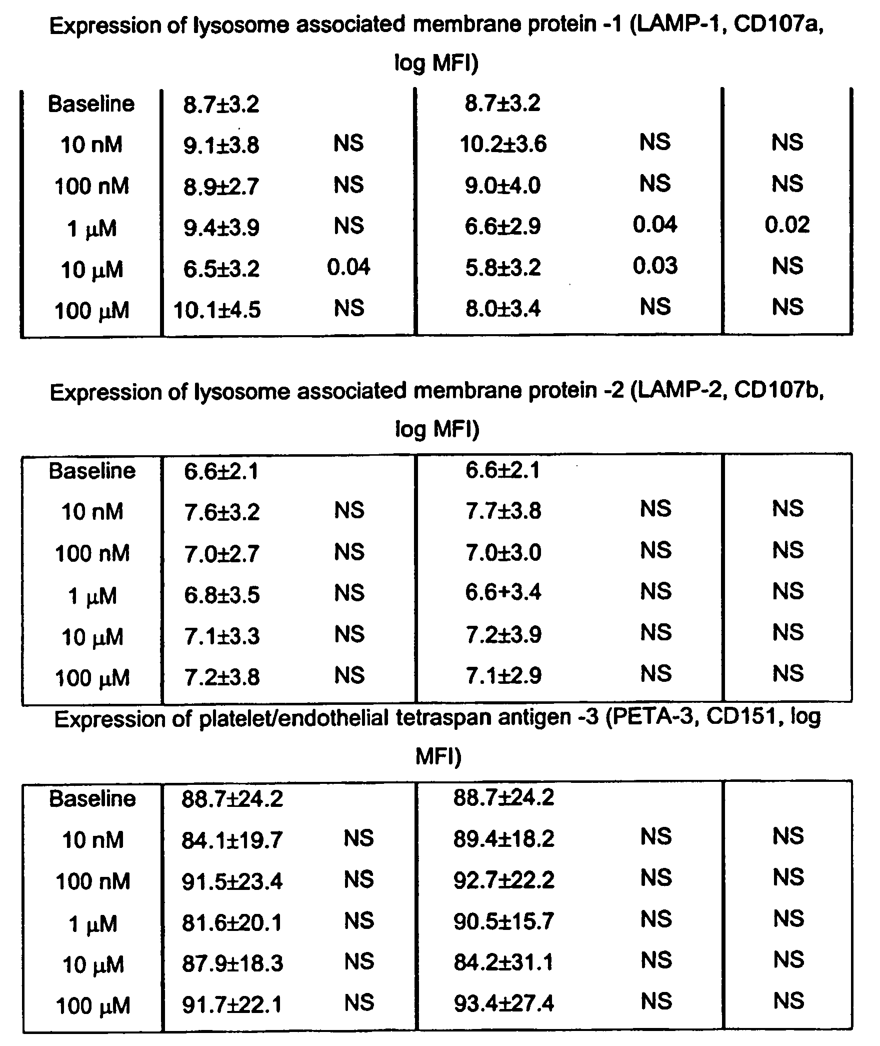

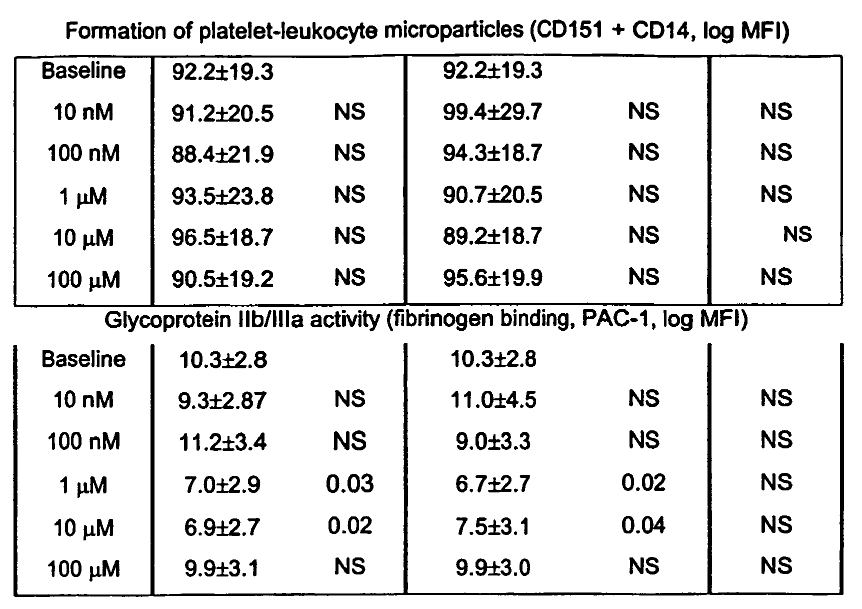

The following examples illustrate the above-described invention; however, it is not intended to restrict the scope of this invention in any manner.

### Example 1

### Method of synthesis of valsartan metabolite

### (S)-2-{(4-Hydroxy-pentanoyl)-[2'-(1H-tetrazol-5-yl)-biphenyl-4-ylmethyl]amino}-3-methyl-butyric acid

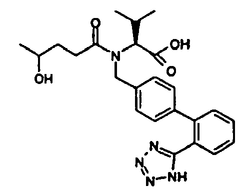

6.7 g (S)-3-Methyl-2-{(4-oxo-pentanoyl)-[2'-(1H-tetrazol-5-yl)-biphenyl-4-ylmethyl]-amino}-butyric acid are dissolved in 60 ml methanol and cooled to 0°C. 2.25 g sodium borohydride are added in small portions in order to keep the stirred reaction mixture below 27°C (strong foaming). The mixture is stirred at room temperature for 1 hour, concentrated in vacuo, dissolved in methylenechloride and extracted twice with 2N aqueous hydrochloric acid. The organic phase is dried, concentrated in vacuo and the product is received by chromatography (flash column, 240 g silicagel 60, KG40-62 micrometer, using a solvent mixture of methylenechloride, methanol, conc. aqueous ammonia (30:10:1 v/v). The fractions containing the product were concentrated, dissolved in methylenechloride and extracted with 2N aqueous hydrochloric acid and dried over sodium sulfate. After concentrating the residue is dried in vacuo (60°C) for 3 days yielding (S)-2-{(4-Hydroxy-pentanoyl)-[2'-(1H-tetrazol-5-yl)-biphenyl-4-ylmethyl]amino}-3-methyl-butyric acid as a white foam ([α]_{D}²⁰=-58° (c=1, methanol). TLC-Rf: 0.18 (toluene/ethylacetate/methylenechloride/formic acid 16:40:40:4).

The starting material (S)-3-Methyl-2-{(4-oxo-pentanoyl)-[2'-(1H-tetrazol-5-yl)-biphenyl-4-ylmethyl]-amino}-butyric acid can be prepared as follows:

### (S)-2-{(2'-Cyano-biphenyl-4-ylmethyl)-[3-(2-methy)-[1,3]dioxolan-2-yl)-propionyl]-amino}. 3-methyl-butyric acid benzyl ester

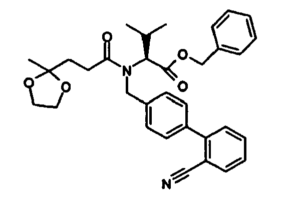

13.8 g (S)-2-[(2'-Cyano-biphenyl-4-ylmethyl)-amino]-3-methyl-butyric acid benzyl ester Hydrochloride (described in EP 443983) are dissolved in 50 ml methylenechloride, cooled to 0°C and treated with 23.8 ml ethyldiisopropylamine (Hünig base). To this mixture is added at 0°C a solution of 3-(2-Methyl-[1,3]dioxolan-2-yl)-propionyl chloride, prepared from 8.9 g 3-(2-Methyl-[1,3]dioxolan-2-yl)-propionic acid (Tetrahedron 37, 307,1981) and 10.31 ml (1-Chloro-2-methyl-propenyl)-dimethyl-amine (Tetrahydron 54, 9207, 1998) in 40 ml methylenechloride. The reaction mixture is stirred at room temperature for 3-4 days, depending on the progress of the transformation. Preferably, 3-(2-Methyl-[1,3]dioxolan-2-yl)-propionyl chloride is added in 3-4 portions over 2 days. The reaction mixture is concentrated in vacuo, dissolved in ethylacetate, washed with water, 1N aqueous hydrochloric acid, water, dried over sodium sulfate and concentrated in vacuo. Flash column chromatography (240 g silicagel 60, 40-63 micrometer, petroleum ether/ethylacetate 2:1 to 1:1) provides, after drying of the product in vacuo at 50 0°C, pure (S)-2-((2-Cyano-biphenyl-4-ylmethyl)-13-(2-methyl-[1,3]dioxolan-2-yl)-propionyo-amino)-3-methyl-butyric acid benzyl ester as a golden, sticky residue. TLC-Rf: 0.23 (petroleum ether/ethylacetate 2:1).

### (S)-2-[(2'-Cyano-biphenyl-4-ylmethyl)-(4-oxo-pentanoyl)-amino]-3-methyl-butyric acid benzyl ester

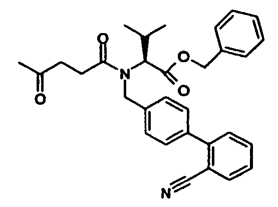

9.8 g (S)-2-((2-Cyano-biphenyl-4-ylmethyl)-[3-(2-methyl-[1,3]dioxolan-2-yl)-propionyl]-amino)-3-methyl-butyric acid benzyl ester are dissolved in 100 ml tetrahydrofurane and treated with 50 ml 1 N aqueous hydrochloric acid. The mixture is stirred at room temperature for 6.5 hours, concentrated in vacuo and extracted with methylenechloride. The organic phase is washed with water, dried over sodium sulfate, concentrated in vacuo, evaporated and dried in vacuo at 50 0°C for 1 hour. (S)-2-[(2'-Cyano-biphenyl-4-ylmethyl)-(4-oxo-pentanoyl)-amino]-3-methyl-butyric acid benzyl ester is received as orange, viscous oil. THL-RF: 0.18 (petroleum ether/ethylacetate 2:1).

### (S)-3-Methyl-2-{(4-oxo-pentanoyl)-[2'-(1H-tetrazol-5-yl)-biphenyl-4 ylmethyl]-amino}-butyric acid benzyl ester

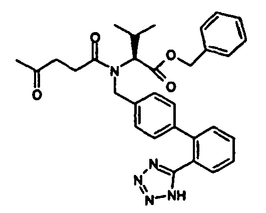

8.64 g (S)-2-[(2'-Cyano-biphenyl-4-ylmethyl)-(4-oxo-pentanoyl)-amino]-3-methyl-butyric acid benzyl ester and 12.71 g Tributyltinazid (Aldrich) in 20 ml xylene are refluxed for 28 hours. The mixture is treated with 0.5N aqueous sodium hydroxide solution, the waterphase is washed with ether and ether phase is extracted once with water. The combined water phase is acidified with concentrated aqueous hydrochloric acid, extracted with methylenechloride, washed with water, suspended with active carbon, filtered, dried over sodium sulfate, concentrated and dried in vacuo. The product (S)-3-Methyl-2-{(4-oxo-pentanoyl)-[2'-(1 H-tetrazol-5-yl)-biphenyl-4-ylmethyl]-amino}-butyric acid benzyl ester is received as brown foam. TLC-Rf: 0.36 (toluene/methylenechloride/methanol/formic acid 40:40:40:4).

### (S)-3-Methyl-2-{(4-oxo-pentanoyl)-[2'-(1 H-tetrazol-5-yl)-biphenyl-4-ylmethyl]-amino}-butyric acid

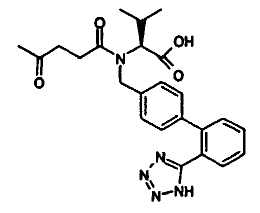

7.9 g (S)-3-Methyl-2-{(4-oxo-pentanoyl)-[2'-(1H-tetrazol-5-yl)-biphenyl-4-ylmethyl]-amino}-butyric acid benzyl ester in 160 ml tetrahydrofuran are hydrogenated under normal pressure at room temperature in the presence of 1.5 g palladium on carbon (10%) until saturation is achieved. The mixture is filtered and concentrated in vacuo providing (S)-3-Methyl-2-{(4-oxo-pentanoyl)-[2'-(1H-tetrazol-5-yl)-biphenyl-4-ylmethyl]-amino}-byutyric acid as an almost white foam. TLC-Rf: 0.1 (toluene/methylenechloride/methanol/formic acid 40:40:40:4).

## Claims

1. Use of the metabolite valeryl 4-hydroxy valsartan or a pharmaceutically acceptable salt thereof for the manufacture of a medicament for inhibiting platelet aggregation, optionally in the presence of a pharmaceutically acceptable carrier.

2. Use of the metabolite valeryl 4-hydroxy valsartan or a pharmaceutically acceptable salt thereof for the manufacture of a medicament for the prevention, delay of progression or treatment of conditions mediated by platelet aggregation.

3. Use of the metabolite valeryl 4-hydroxy valsartan or a pharmaceutically acceptable salt thereof for the manufacture of a medicament for the prevention, delay of progression or treatment of a disease and disorder mediated by platelet aggregation, in particular, acute myocardial infarction, ischemic stroke, angina pectoris, acute coronary syndromes, TIA (transient ischemic attacks, or acute cerebrovascular syndromes), heart failure, chest pain of ischemic etiology, syndrome X, thromboembolism, pulmonary hypertension, diabetes mellitus, peripheral vascular disease, deep vein thrombosis, arterial thrombosis of any vessel, catheter thrombotic occlusion or reocclusion.

4. The metabolite valeryl 4-hydroxy valsartan or a pharmaceutically acceptable salt thereof for use as a medicament

## Patentansprüche

1. Verwendung des Metaboliten Valeryl-4-hydroxyvalsartan oder eines pharmazeutisch annehmbaren Salzes hiervon zur Herstellung eines Arzneimittels zur Hemmung der Blutplättchenaggregation, wahlweise in Gegenwart eines pharmazeutisch annehmbaren Trägers.

2. Verwendung des Metaboliten Valeryl-4-hydroxyvalsartan oder eines pharmazeutisch annehmbaren Salzes hiervon zur Herstellung eines Arzneimittels zur Prävention, Progressionsverzögerung oder Behandlung von Zuständen, die durch Blutplättchenaggregation vermittelt werden.

3. Verwendung des Metaboliten Valeryl-4-hydroxyvalsartan oder eines pharmazeutisch annehmbaren Salzes hiervon zur Herstellung eines Arzneimittels zur Prävention, Progressionsverzögerung oder Behandlung einer Erkrankung oder eines Zustands, der durch Blutplättchenaggregation vermittelt wird, insbesondere akuter Myokardinfarkt, ischämischer Schlaganfall, Angina pektoris, akute Koronarsyndrome, TIA (transiente ischämische Attacken oder akute cerebrovaskuläre Syndrome), Herzversagen, Brustschmerz ischämischer Ätiologie, Syndrom X, Thromboembolie, pulmonaler Hypertension, Diabetes mellitus, periphere vaskuläre Erkrankung, tiefe Venenthrombose, arterielle Thrombose eines Gefäßes, thrombotischer Katheterverschluss oder Wiederverschluss.

4. Metabolit Valeryl-4-hydroxyvalsartan oder ein pharmazeutisch annehmbares Salz hiervon zur Verwendung als Arzneimittel.

## Revendications

1. Utilisation du métabolite valéryl 4-hydroxy valsartan ou d'un de ses sels pharmaceutiquement acceptables, dans la fabrication d'un médicament destiné à inhiber l'agrégation plaquettaire, éventuellement en présence d'un véhicule pharmaceutiquement acceptable.

2. Utilisation du métabolite valéryl 4-hydroxy valsartan ou d'un de ses sels pharmaceutiquement acceptables, dans la fabrication d'un médicament destiné à la prévention, au ralentissement de la progression ou au traitement de maladies provoquées par l'intermédiaire de l'agrégation plaquettaire.

3. Utilisation du métabolite valéryl 4-hydroxy valsartan ou d'un de ses sels pharmaceutiquement acceptables, dans la fabrication d'un médicament destiné à la prévention, au ralentissement de la progression ou au traitement d'une maladie et d'un trouble provoqués par l'intermédiaire de l'agrégation plaquettaire, tout particulièrement, l'infarctus du myocarde aigu, l'accident ischémique cérébral, l'angor, les syndromes coronariens aigus, les AIT (accidents ischémiques transitoires ou les syndromes cérébrovasculaires aigus), l'insuffisance cardiaque, la douleur thoracique ayant une étiologie ischémique, le syndrome X, la thromboembolémie, l'hypertension pulmonaire, le diabète juvénile, les maladies vasculaires périphériques, la thrombose veineuse profonde, la thrombose artérielle affectant n'importe quel vaisseau, l'occlusion thrombotique du cathéter ou la réocclusion.

4. Métabolite valéryl 4-hydroxy valsartan, ou d'un de ses sels pharmaceutiquement acceptables, utilisable en tant que médicament.
